# EUROPEAN PATENT SPECIFICATION

(11) **EP 4 144 283 B1**
(45) Date of publication and mention of the grant of the patent: **04.09.2024**
(21) Application number: 22192789.0
(22) Date of filing: 30.08.2022
(51) Int. Cl.: A47L 23/26

(54) **SANITIZING MAT**
DESINFEKTIONSMATTE
TAPIS DE DÉSINFECTION

(30) Priority: 01.09.2021 IT 202100022649
(43) Date of publication of application: 08.03.2023
(73) Proprietor: Borin Line S.r.l., 37058 Sanguinetto (VR) (IT)
(72) Inventor: BORIN, Matteo, I-37058 Sanguinetto, VERONA (IT); CODOGNOLA, Nicola, I-37058 Sanguinetto, VERONA (IT)
(74) Representative: Mitola, Marco

(56) References cited:
- WO-A1-03/092747
- WO-A1-2019/155290
- FR-A7- 2 284 252
- IT-A1- UB20 155 757
- US-A1- 2019 133 414

## Description

The present invention lies in the technical field related to floor cleaning devices that are suitable for being placed at the entrance of production areas or of residential or commercial buildings where a high level of cleaning and/or sanitization at the access is required.

In particular, the object of the present invention is a sanitizing mat and a sanitization kit for cleaning soles and/or wheels.

Because of the current pandemic situation due to the spread of the SARS-CoV2 virus, the need to ensure correct sanitization of environments has increased substantially. This need, already perceived in the past and exacerbated by the spread of the COVID-19 disease, has favored the use of floor cleaning devices for the correct cleansing of soles and/or wheels, for example wheels of lift trucks or of generic means of transportation, before they enter an environment such as a production area, a commercial building or a residential structure.

In the prior art, devices constituted by a tank made of a metal material where a grill is housed are already known. Clumps of bristles protrude through the openings of the grill and, when a means of transport or a user passes, interfere with the wheels or with the soles in such a way as to remove dust, dirt and/or incrustations which, once removed, are deposited in the bottom of the tank.

Disadvantageously, because of the grill and the clumps of bristles, it is difficult to determine from the outside the amount of dust, dirt and incrustations that has collected in the bottom of the tank. Therefore, a maintenance operator should raise the grill in order to inspect the tank and, when necessary, vacuum out the dust, dirt and incrustations. Considering the heavy weight of the grill, it is easy to understand how the maintenance operations are extremely inconvenient and laborious for the operator.

The patent 102015000075081, filed in the name of the same Applicant, describes a floor cleaning device equipped with a tank and removable brushing modules. Each brushing module comprises a stainless steel sheet on which a tile made of POM, that is polyoxymethylene, is fixed, from which groups of bristles protrude. The metal sheet applied to the tile ensures dimensional stability and rigidity, so as to avoid thermal expansion and permanent deformations of the brushing module.

Disadvantageously, as far as each brushing module is individually removable, the presence of the metal sheet makes the tile heavy, and therefore the maintenance operations, however simplified, are still burdensome for the operator.

The object of the present invention is to propose a sanitizing mat and a sanitization kit for cleaning soles and/or wheels that is capable of overcoming, at least in part, the aforementioned drawbacks related to the floor cleaning devices of the prior art.

The document IT UB20 155 757 A1 describes a sanitizing mat according to the preamble of independent claim 1.

US 2019/133414 A1 shows a sanitizing device in the form of a treatable mat, in particular a doormat, having the function of sanitizing the support surfaces of a user on the mat, in particular the soles of the shoes.

FR 2 284 252 A7 describes doormat consists of a frame with a set of supports in which the bristles are inset in strips, in slots in the supports, with the strips at right angles to the direction of movement of the feet of the user.

In particular, it is the object of the present invention to propose a sanitizing mat that is able to carry out effective cleaning of the wheels of a generic means of transport, for example a forklift or a lift truck, or of the soles of a user.

A further object of the present invention is to obtain a floor cleaning device that is capable of combining the high cleaning effect with a structure that is simpler and easier to manage.

These objects are achieved by a sanitizing mat according to claim 1 and by a sanitization kit according to claim 13. The dependent claims describe preferred embodiments of the invention.

The features and the advantages of the sanitizing mat and of the sanitization kit according to the invention shall be made readily apparent from the following description of preferred embodiments thereof, provided purely by way of indicative and non-limiting example, with reference to the accompanying figures, wherein:
- Figure 1 is a perspective view of a sanitizing mat in one embodiment;
- Figure 2 is a perspective view of a sanitizing module;

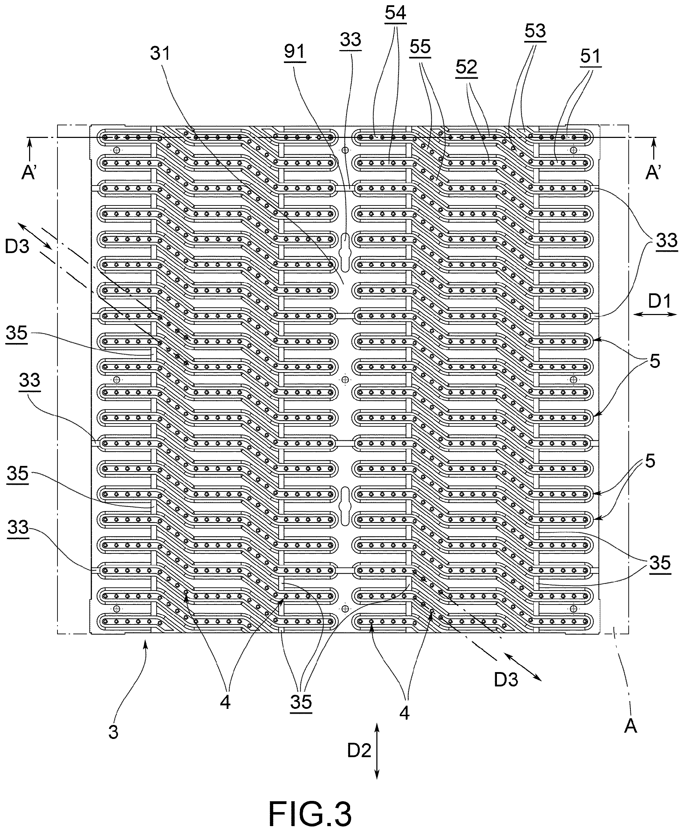

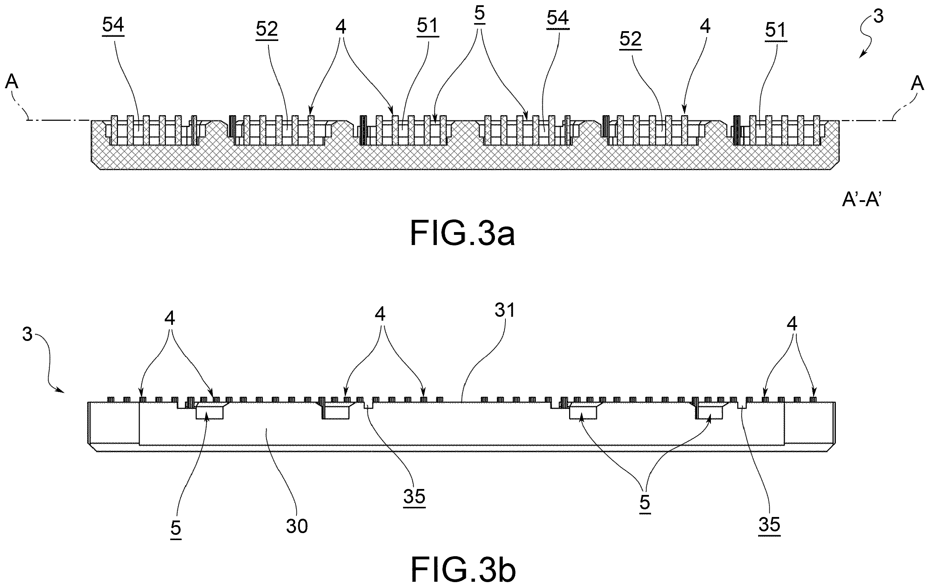

- Figure 3 is a plan view from above of the sanitizing module of Figure 2;
- Figure 3a is a sectional view along line A'-A' of Figure 3;
- Figure 3b is a front view of the sanitizing module of Figure 3;

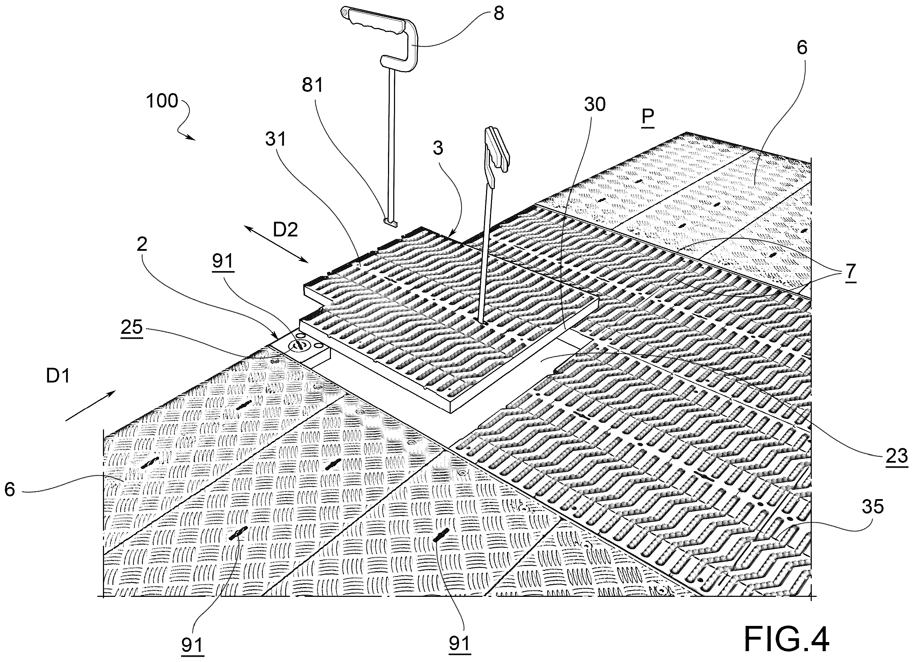

- Figure 4 is a perspective view of a sanitization kit in one embodiment;
- Figure 5 is a perspective, partially exploded view of a detail of the sanitization kit of Figure 4;

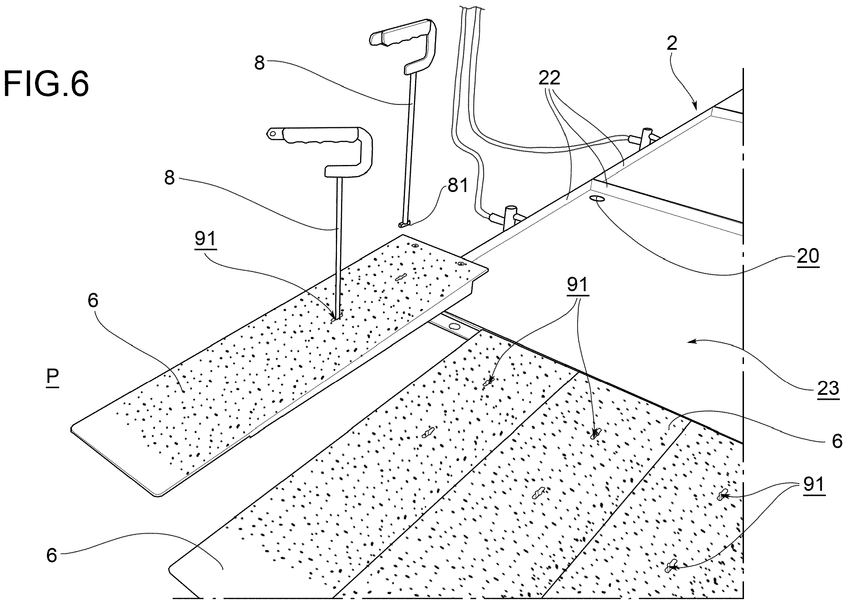

- Figure 6 is a perspective view of partially separated components of a tank and of a plurality of ramp modules; and
- Figure 7 is a perspective view of separated components of the tank in one embodiment.

In the following description, elements common to the various embodiments represented in the drawings are indicated with the same reference numerals.

In said drawings, a sanitizing mat has been indicated with 1 and a sanitization kit has been indicated with 100 according to the invention as a whole.

In a general embodiment, the sanitizing mat 1 for cleaning soles and/or wheels is suitable for being embedded in a walking surface P or placed on said walking surface P.

The sanitizing mat 1 comprises a tank 2 and at least one sanitizing module 3 which may be housed in the tank 2.

The sanitizing module 3 comprises in turn a support base 30 and a plurality of bristle bundles 4. The support base 30 is delimited above by a support surface 31 lying on an imaginary support plane A.

For the purposes of the present discussion, when the sanitizing mat 1 lies on the walking surface P, if the term "upper" (and related derivatives) is associated with a characteristic (or with an element), it is to be understood that said characteristic (or element) is positioned distally with respect to the walking surface P. Vice versa, when the term "lower" (and related derivatives) is associated with a characteristic (or with an element), it is to be understood that said characteristic (or element) is positioned proximally with respect to the walking surface P.

Furthermore, considering the sanitizing mat 1 in operational conditions, that is embedded in or rested on the walking surface P so that it may be walked on with a sole and/or traveled over with a wheel, the direction orthogonal to the walking surface defines a vertical direction.

A plurality of slits 5 is made in the support base 30 and each slit of said plurality of slits 5 is configured as a depression with respect to the imaginary support plane A.

Each slit further comprises a first groove 51 and a second groove 52 arranged parallel to each other along a first direction of use D1 and connected by a connecting groove 53 incident to the first direction of use.

Bundles of bristles are fixed at the bottom of each slit, wherein each bristle bundle 4 of said plurality of bristle bundles 4 projects beyond the lying surface of the imaginary support plane A to interfere with the soles or wheels and to exert a cleaning action of brushing or scrubbing.

In other words, the cleaning is guaranteed by the mechanical scrubbing action of the bristle bundles against the contact surface of the wheel or of the sole.

The width of the slit is greater than that of each bristle bundle so as to allow the possible suction of dirt and debris accumulated at the bottom of the slit.

It is specified that, in the case of the first groove 51 and of the second groove 52, the width of the slit is measured orthogonally to the first direction of use D1. In the case of the connecting groove 53, however, the width of the slit is measured orthogonally to the main direction of the development of the connecting groove 53.

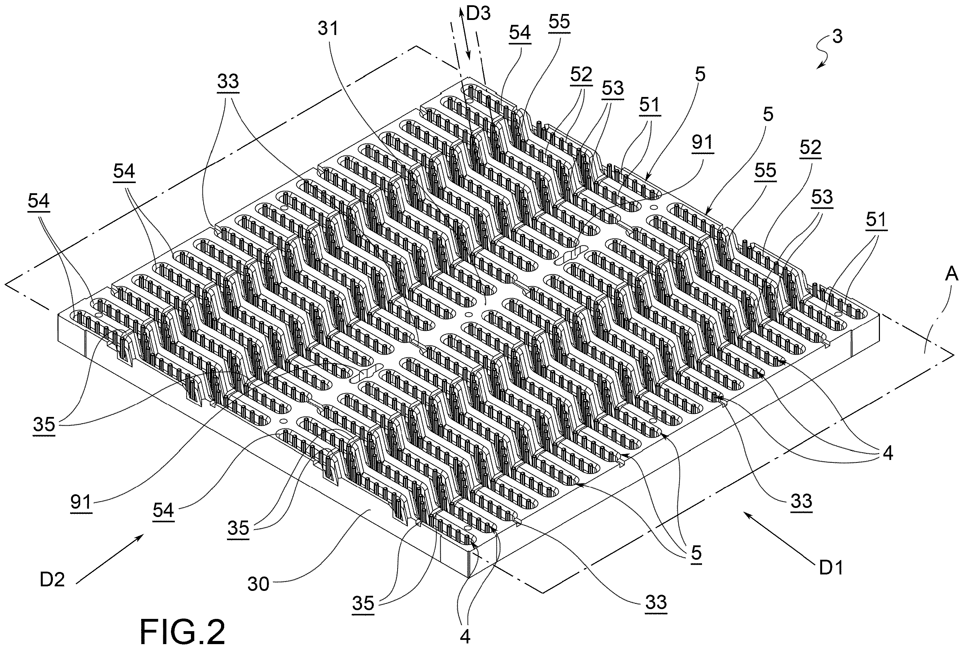

In one embodiment, each slit comprises at least one further groove 54 arranged along the first direction of use D1 and connected to the first groove 51 or to the second groove 52 by means of at least one further connecting groove 55 (Figure 2).

According to an embodiment, the connecting groove 53 and the further connecting groove 55 are arranged parallel to each other along a connecting direction D3 incident to the first direction of use D1.

Furthermore, the connecting groove 53 connects an end of the first groove 51 to an end of the second groove 52; while the further connecting groove 55 connects an end of the further groove 54 to an end of the first 51 or second groove 52.

According to one embodiment, the sanitizing mat 1 also comprises a cleaning solution contained in the tank 2.

Thanks to the presence of the cleaning solution, the cleaning efficacy due to the mechanical scrubbing action of the bristle bundles is further increased.

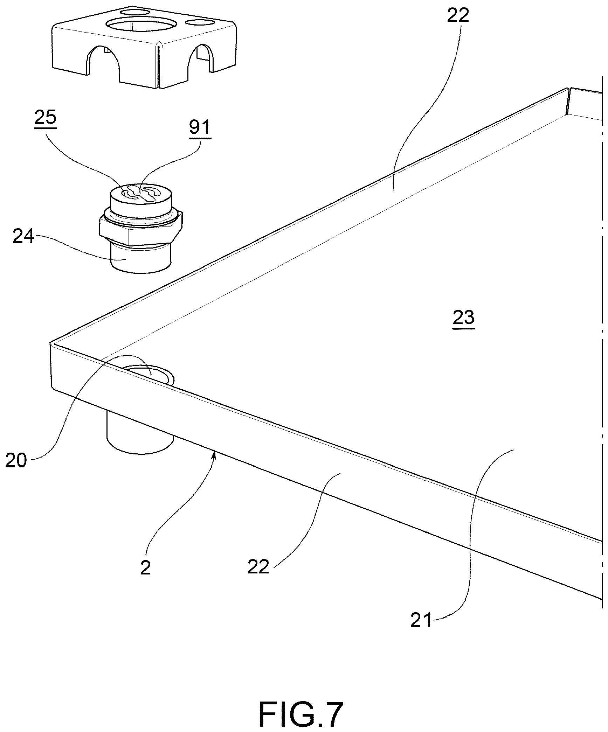

As shown in the embodiment of Figure 7, a drainage hole 20 is made in the tank 2 for draining any fluid collected in the tank 2, or for suctioning debris that has accumulated at the bottom of the tank.

Preferably, the tank 2 is made of stainless steel.

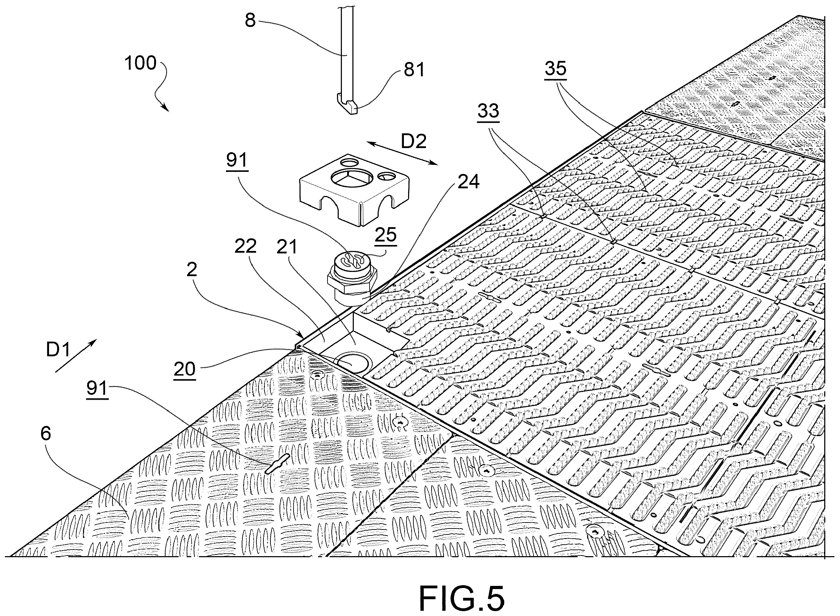

According to the embodiment shown in the attached Figures 5 and 7, the tank 2 is defined by a bottom wall 21 and a side wall 22 delimiting a compartment 23. The drainage hole 20 is obtained in the bottom wall 21.

The tank 2 further comprises an extension 24 engageable to the drainage hole 20 so as to protrude from the bottom wall 21. This extension has at least one opening 25 in fluidic communication with the drainage hole 20 and suitable for preventing the cleaning solution or any fluid collected in the tank 2 from exceeding a predetermined limit level.

For example, during the use of the sanitizing mat, the volume occupied by the sole of a user or by the wheel of a generic means of transport causes a raising of the level of cleaning solution which, as a result, may exceed the predetermined threshold level. The opening 25, which is configured as an upper spillway, prevents the tank 2 from overflowing.

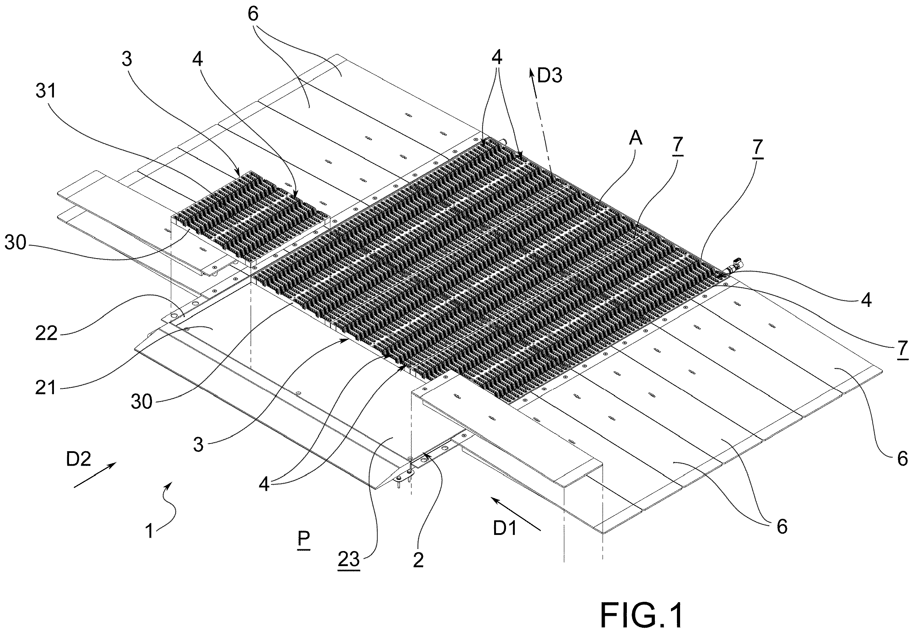

According to the embodiment of Figure 1, a plurality of sanitizing modules placed side by side so that the support surface 31 of each sanitizing module 3 lies on the imaginary support plane A is housed in the tank 2. In the present case, the plurality of sanitizing modules is arranged so as to completely occupy the compartment 23 of the tank 2. Furthermore, said sanitizing modules are placed side by side with play in between them.

The level of cleaning solution contained in the tank 2 exceeds the lay of the imaginary support plane A, so that only the plurality of bristle bundles 4 projects beyond the lying plane of said imaginary support plane A. In other words, with the exception of the plurality of bristle bundles 4, the plurality of sanitizing modules turns out to be substantially immersed in the cleaning solution.

For the purposes of the present description, it is specified that the predetermined threshold level and the level of cleaning solution are measured along the vertical direction.

In one embodiment, the opening 25 is positioned at a height, measured vertically starting from the walking surface P, which is higher than the height at which the imaginary support plane A lies.

According to one embodiment, each sanitizing module 3 is tile-shaped and bears a grid of channels 33, 35 for channeling the cleaning solution. Said grid of channels 33, 35 is also in fluidic communication with the plurality of slits 5, so that each bristle bundle, subjected to the weight force exerted by a sole or by a wheel, flattens getting wet in the cleaning solution and exerting both a cleaning and cleansing action by brushing and/or scrubbing.

In particular, the "tile" shape is substantially a parallelepiped shape in which two dimensions are preponderant over a third one.

In one embodiment, the grid of channels is obtained on the support surface 31 of each sanitizing module 3 and comprises a first group of channels 33 oriented parallel to each other along the first direction of use D1 and/or a second group of channels 35 arranged parallel to each other and oriented along a second direction of use D2 incident to the first D1.

Preferably, said first D1 and second directions of use D2 are orthogonal to each other.

Advantageously, the first and the second direction of use define two main directions of use of the sanitizing mat where the plurality of bristle bundles carries out the greatest brushing and/or scrubbing cleaning action, ensuring maximum cleaning and cleansing.

According to one embodiment, between one sanitizing module 3 and the adjacent sanitizing module, and/or between a sanitizing module 3 and the side wall 22 of the tank 2, there is a gap 7 allowing the cleaning solution to reach the grid of channels 33, 35 and the plurality of slits 5.

According to one embodiment, the support base 30 is made of POM-C.

Advantageously, the polyacetal resin (or POM-C) is a semi-crystalline copolymer which has a density greater than that of POM, thereby increases the rigidity of the sanitizing module and reduces the risk of undesirable permanent deformations.

A further advantage due to the fact that the sanitizing modules are made of POM-C is that of making the presence of the stainless steel plate no longer necessary, and therefore every sanitizing module turns out to be lighter and easily maneuverable by the operator.

According to one aspect of the invention, the sanitization kit 100 for cleaning soles and/or wheels comprising a sanitizing mat 1 and plurality of ramp modules 6 is proposed, wherein each ramp module of said plurality of ramp modules 6 is an inclined plane for connecting the walking surface P with the imaginary support plane A.

Preferably, each ramp module is made of polyethylene or stainless steel.

In one embodiment, the sanitization kit 100 further comprises a lifting and release tool 8 provided with coupling means 81 engageable with respective coupling counter-means 91 obtained on each sanitization module 3, on each ramp module of the plurality of ramp modules 6 and/or on the extension 24.

Innovatively, the sanitizing mat and the sanitization kit according to the present invention achieve the aforementioned object.

Advantageously, the sanitizing mat that is the object of the present invention is extremely versatile, as it may be utilized dry or damp, i.e., with the cleaning solution.

According to one advantageous aspect, the sanitization kit allows the use of the sanitizing mat on any type of walking surface without the need to perform any masonry installation work on the ground for the mat.

According to a yet another advantageous aspect, the sanitizing mat that is the object of the present invention requires minimal maintenance, because when used dry it is sufficient to suction the residues of dust, dirt and incrustations from the bottom of the slits, whereas, when used damp, it is sufficient to drain the cleaning solution via the drainage hole.

## Claims

1. Sanitizing mat (1) for cleaning soles and/or wheels suitable for being embedded in or placed on a walking surface (P), said sanitizing mat comprising:
- a tank (2);
- at least one sanitizing module (3) housed in the tank (2) and comprising a support base (30) delimited above by a support surface (31) lying on an imaginary support plane (A), and a plurality of bristle bundles (4), **characterized in that**
a plurality of slits (5) is made in the support base (30), wherein each slit of said plurality of slits (5) is configured as a depression with respect to the imaginary support plane (A) and comprises a first groove (51) and a second groove (52) arranged parallel to each other along a first direction of use (D1) and connected by a connecting groove (53) incident to the first direction of use,
at the bottom of each slit bundles of bristles being fixed, wherein each bundle of bristles of said plurality of bundles of bristles (4) projects beyond the lying surface of the imaginary support plane (A) to interfere with the soles or wheels and to exert a cleaning action of brushing or scrubbing,
and wherein the width of the slit is greater than that of each bundle of bristles so as to allow the possible suction of dirt and debris accumulated at the bottom of the slit.

2. Sanitizing mat (1) according to the preceding claim, wherein each slit comprises at least one further groove (54) arranged along the first direction of use (D1) and connected to the first groove (51) or to the second groove (52) by means of at least one further connecting groove (55).

3. Sanitizing mat (1) according to the preceding claim, wherein the connecting groove (53) and the further connecting groove (55) are arranged parallel to each other along a connecting direction (D3) incident to the first direction of use (D1), and
wherein the connecting groove (53) connects an end of the first groove (51) to an end of the second groove (52), and
the further connecting groove (55) connects an end of the further groove (54) to an end of the first (51) or second groove (52).

4. Sanitizing mat (1) according to any of the preceding claims, comprising a cleaning solution contained in the tank (2).

5. Sanitizing mat (1) according to any of the preceding claims, wherein a drainage hole (20) is also made in the tank (2) for draining any fluid collected in the tank (2), or for suctioning debris that has accumulated at the bottom of the tank.

6. Sanitizing mat (1) according to claim 4 and 5, wherein the tank (2) is defined by a bottom wall (21) and a side wall (22) delimiting a compartment (23), in the bottom wall (21) the drain hole (20) being made, and wherein the tank (2) further comprises an extension (24) engageable to the drain hole (20) so as to protrude from the bottom wall (21), said extension having at least one opening (25) in fluidic communication with the drain hole (20) and suitable to prevent the detergent solution or any fluid collected in the tank (2) from exceeding a predetermined limit level.

7. Sanitizing mat (1) according to any of the claims from 4 to 6, wherein the tank (2) houses a plurality of sanitizing modules placed side by side so that the support surface (31) of each sanitizing module (3) lies on the imaginary support plane (A), and
wherein the level of detergent solution contained in the tank (2) exceeds the height of the imaginary support plane (A), so that only the plurality of bristle bundles (4) projects beyond the lying plane of said imaginary support plane (A).

8. Sanitizing mat (1) according to claims 6 and 7, wherein the opening (25) is positioned at a height, measured vertically starting from the walking surface (P), which is higher than the height at which the imaginary support plane (A) lies.

9. Sanitizing mat (1) according to any of the claims from 4 to 8, wherein each sanitizing module (3) is tile-shaped and bears a grid of channels (33, 35) for channelling the detergent solution, said grid of channels (33, 35) also being in fluidic communication with the plurality of slits (5), so that each bundle of bristles, subjected to the weight force exerted by a sole or a wheel, is flattened and wetted in the detergent solution and exerts both a cleaning and a cleansing action by brushing and/or scrubbing.

10. Sanitizing mat (1) according to the preceding claim, wherein the grid of channels is made on the support surface (31) of each sanitizing module (3) and comprises a first group of channels (33) oriented parallel to each other along the first direction of use (D1) and/or a second group of channels (35) arranged parallel to each other and oriented along a second direction of use (D2) incident to the first (D1); preferably said first (D1) and second directions of use (D2) being orthogonal to each other.

11. Sanitizing mat (1) according to claim 9 or 10, wherein between one sanitizing module (3) and the adjacent sanitizing module, and/or between a sanitizing module (3) and the side wall (22) of the tank (2), there is a gap (7) allowing the cleaning solution to reach the grid of channels (33, 35) and the plurality of slits (5).

12. Sanitizing mat (1) according to any of the preceding claims, wherein the support base (30) is made of POM-C.

13. Sanitization kit (100) for cleaning soles and/or wheels comprising a sanitizing mat (1) according to any of the claims from 1 to 12 and a plurality of ramp modules (6), wherein each ramp module of said plurality of ramp modules (6) is an inclined plane for connecting the walking surface (P) with the imaginary support plane (A) .

14. Sanitization kit (100) according to the preceding claim in combination with claim 6, further comprising a lifting and release tool (8) provided with coupling means (81) engageable with respective coupling counter-means (91) made on each sanitization module (3), on each ramp module of the plurality of ramp modules (6) and/or on the extension (24).

## Patentansprüche

1. Desinfektionsmatte (1) zum Reinigen von Sohlen und/oder Rädern, die dazu geeignet ist, in eine Lauffläche (P) eingebettet oder auf dieser platziert zu werden, wobei die Desinfektionsmatte aufweist:
- einen Behälter (2);
- zumindest ein Desinfektionsmodul (3), das in dem Behälter (2) aufgenommen ist und eine Stützbasis (30), die oben durch eine auf einer gedachten Auflageebene (A) liegende Auflagefläche (31) begrenzt ist, und eine Vielzahl von Borstenbündeln (4) aufweist,
**dadurch gekennzeichnet, dass**
eine Vielzahl von Schlitzen (5) in der Stützbasis (30) ausgebildet ist, wobei jeder Schlitz der Vielzahl von Schlitzen (5) als Vertiefung in Bezug auf die imaginäre Auflageebene (A) ausgebildet ist und eine erste Nut (51) und eine zweite Nut (52) aufweist, die parallel zueinander entlang einer ersten Verwendungsrichtung (Dl) angeordnet und durch eine Verbindungsnut (53) verbunden sind, die in der ersten Verwendungsrichtung verläuft,
wobei an der Bodenseite jedes Schlitzes Borstenbündel befestigt sind, wobei jedes Borstenbündel der Vielzahl von Borstenbündeln (4) über die Liegefläche der gedachten Auflageebene (A) hinausragt, um mit den Sohlen oder Rädern zu interferieren und eine Reinigungswirkung (Bürsten oder Schrubben) auszuüben,
und wobei die Breite des Schlitzes größer ist als jene jedes Borstenbündels, um das mögliche Aufsaugen von Schmutz und Ablagerungen zu ermöglichen, die sich am Boden des Schlitzes angesammelt haben.

2. Desinfektionsmatte (1) nach dem vorstehenden Anspruch, wobei jeder Schlitz zumindest eine weitere Nut (54) aufweist, die entlang der ersten Verwendungsrichtung (Dl) angeordnet ist und über zumindest eine weitere Verbindungsnut (55) mit der ersten Nut (51) oder mit der zweiten Nut (52) verbunden ist.

3. Desinfektionsmatte (1) nach dem vorstehenden Anspruch, wobei die Verbindungsnut (53) und die weitere Verbindungsnut (55) entlang einer Verbindungsrichtung (D3), die auf die erste Verwendungsrichtung (D1) trifft, parallel zueinander angeordnet sind, und
wobei die Verbindungsnut (53) ein Ende der ersten Nut (51) mit einem Ende der zweiten Nut (52) verbindet, und
die weitere Verbindungsnut (55) ein Ende der weiteren Nut (54) mit einem Ende der ersten (51) oder zweiten Nut (52) verbindet.

4. Desinfektionsmatte (1) nach einem der vorstehenden Ansprüche, aufweisend eine in dem Behälter (2) enthaltene Reinigungslösung.

5. Desinfektionsmatte (1) nach einem der vorstehenden Ansprüche, wobei in dem Behälter (2) ebenfalls ein Ablaufloch (20) vorgesehen ist, um in dem Behälter (2) angesammelte Flüssigkeit abzulassen oder um an dem Boden des Behälters angesammelten Schmutz abzusaugen.

6. Desinfektionsmatte (1) nach Anspruch 4 und 5, wobei der Behälter (2) durch eine Bodenwand (21) und eine Seitenwand (22) definiert ist, die eine Kammer (23) begrenzen, wobei in der Bodenwand (21) das Ablaufloch (20) ausgebildet ist, und wobei der Behälter (2) ferner eine Verlängerung (24) aufweist, die mit dem Ablaufloch (20) in Eingriff gebracht werden kann, so dass sie von der Bodenwand (21) vorsteht, wobei die Verlängerung zumindest eine Öffnung (25) aufweist, die in fluidischer Verbindung mit dem Ablaufloch (20) steht und geeignet ist, zu verhindern, dass die Waschmittellösung oder ein Fluid, das sich in dem Behälter (2) angesammelt hat, einen vorgegebenen Grenzpegel überschreitet.

7. Reinigungsmatte (1) nach einem der Ansprüche 4 bis 6, wobei der Behälter (2) eine Vielzahl von Reinigungsmodulen aufnimmt, die derart nebeneinander angeordnet sind, dass die Auflagefläche (31) eines jeden Reinigungsmoduls (3) auf der gedachten Auflageebene (A) liegt, und
wobei der Pegel der in dem Behälter (2) enthaltenen Reinigungslösung die Höhe der gedachten Auflageebene (A) übersteigt, so dass nur die Vielzahl von Borstenbündeln (4) über die Liegeebene der gedachten Auflageebene (A) hinausragt.

8. Desinfektionsmatte (1) nach Anspruch 6 und 7, wobei die Öffnung (25) in einer Höhe angeordnet ist, die, vertikal von der Lauffläche (P) aus gemessen, höher ist als die Höhe, in der die gedachte Auflageebene (A) liegt.

9. Desinfektionsmatte (1) nach einem der Ansprüche 4 bis 8, wobei jedes Reinigungsmodul (3) ein Gitter von Kanälen (33, 35) aufweist und trägt, um die Reinigungslösung zu leiten, wobei das Gitter von Kanälen (33, 35) auch mit der Vielzahl von Schlitzen (5) in fluidischer Kommunikation steht, so dass jedes Borstenbündel, das der von einer Sohle oder einem Rad ausgeübten Gewichtskraft ausgesetzt ist, abgeflacht und in der Reinigungsmittellösung benetzt wird und durch Bürsten und/oder Schrubben sowohl eine Reinigungs- als auch eine Säuberungswirkung ausübt.

10. Desinfektionsmatte (1) nach dem vorstehenden Anspruch, wobei das Gitter von Kanälen auf der Auflagefläche (31) jedes Desinfektionsmoduls (3) ausgebildet ist und eine erste Gruppe von Kanälen (33), die parallel zueinander entlang der ersten Verwendungsrichtung (D1) ausgerichtet sind, und/oder eine zweite Gruppe von Kanälen (35) aufweist, die parallel zueinander angeordnet und entlang einer zweiten Verwendungsrichtung (D2) ausgerichtet sind, die auf die Erste (D1) trifft; wobei die erste (D1) und die zweite Verwendungsrichtung (D2) bevorzugt orthogonal zueinander sind.

11. Desinfektionsmatte (1) nach Anspruch 9 oder 10, wobei zwischen einem Desinfektionsmodul (3) und dem benachbarten Desinfektionsmodul und/oder zwischen einem Desinfektionsmodul (3) und der Seitenwand (22) des Behälters (2) ein Spalt (7) vorhanden ist, der es der Reinigungslösung ermöglicht, zu dem Gitter von Kanälen (33, 35) und der Vielzahl von Schlitzen (5) zu gelangen.

12. Desinfektionsmatte (1) nach einem der vorstehenden Ansprüche, wobei die Stützbasis (30) aus POM-C hergestellt ist.

13. Desinfektionskit (100) zur Reinigung von Sohlen und/oder Rädern, aufweisend eine Desinfektionsmatte (1) nach einem der Ansprüche 1 bis 12 sowie eine Vielzahl von Rampenmodulen (6), wobei jedes Rampenmodul der Vielzahl von Rampenmodulen (6) eine schiefe Ebene zur Verbindung der Lauffläche (P) mit der gedachten Auflageebene (A) ist.

14. Desinfektionskit (100) nach dem vorstehenden Anspruch in Kombination mit Anspruch 6, ferner aufweisend ein Hebe- und Freigabewerkzeug (8), das mit Kopplungsmitteln (81) versehen ist, die mit entsprechenden Kopplungsgegenmitteln (91) in Eingriff bringbar sind, die an jedem Desinfektionsmodul (3), an jedem Rampenmodul der Vielzahl von Rampenmodulen (6) und/oder an der Verlängerung (24) ausgebildet sind.

## Revendications

1. Tapis de désinfection (1) pour nettoyer des semelles et/ou des roues, approprié pour être intégré dans ou placé sur une surface de marche (P), ledit tapis de désinfection comprenant :
- un réservoir (2) ;
- au moins un module de désinfection (3) logé dans le réservoir (2) et comprenant une base de support (30) délimitée au-dessus par une surface de support (31) reposant sur un plan de support imaginaire (A), et une pluralité de faisceaux de poils (4),
**caractérisé en ce qu'**une pluralité de fentes (5) sont constituées dans la base de support (30), dans lequel chaque fente de ladite pluralité de fentes (5) est configurée comme un évidement par rapport au plan de support imaginaire (A) et comprend une première rainure (51) et une deuxième rainure (52) agencées parallèlement l'une à l'autre dans une première direction d'utilisation (D1) et reliées par une rainure de liaison (53) incidente à la première direction d'utilisation,
au fond de chaque fente sont fixés des faisceaux de poils, dans lequel chaque faisceau de poils de ladite pluralité de faisceaux de poils (4) fait saillie au-delà de la surface de repos du plan de support imaginaire (A) pour interférer avec les semelles ou les roues et pour exercer une action de nettoyage de brossage ou de récurage,
et dans lequel la largeur de la fente est supérieure à celle de chaque faisceau de poils de manière à permettre l'aspiration éventuelle de saleté et de débris accumulés au fond de la fente.

2. Tapis de désinfection (1) selon la revendication précédente, dans lequel chaque fente comprend au moins une rainure supplémentaire (54) agencée dans la première direction d'utilisation (D1) et reliée à la première rainure (51) ou à la deuxième rainure (52) au moyen d'au moins une rainure de liaison supplémentaire (55).

3. Tapis de désinfection (1) selon la revendication précédente, dans lequel la rainure de liaison (53) et la rainure de liaison supplémentaire (55) sont agencées parallèlement l'une à l'autre dans une direction de liaison (D3) incidente à la première direction d'utilisation (D1), et
dans lequel la rainure de liaison (53) relie une extrémité de la première rainure (51) à une extrémité de la deuxième rainure (52), et
la rainure de liaison supplémentaire (55) relie une extrémité de la rainure supplémentaire (54) à une extrémité de la première rainure (51) ou de la deuxième rainure (52).

4. Tapis de désinfection (1) selon l'une quelconque des revendications précédentes, comprenant une solution de nettoyage contenue dans le réservoir (2).

5. Tapis de désinfection (1) selon l'une quelconque des revendications précédentes, dans lequel un trou d'évacuation (20) est également constitué dans le réservoir (2) pour évacuer tout fluide collecté dans le réservoir (2), ou pour aspirer des débris qui ont été accumulés au fond du réservoir.

6. Tapis de désinfection (1) selon les revendications 4 et 5, dans lequel le réservoir (2) est défini par une paroi inférieure (21) et une paroi latérale (22) délimitant un compartiment (23), le trou d'évacuation (20) étant constitué dans la paroi inférieure (21), et dans lequel le réservoir (2) comprend en outre une extension (24) pouvant s'engager dans le trou d'évacuation (20) de manière à faire saillie à partir de la paroi inférieure (21), ladite extension comportant au moins une ouverture (25) en communication fluidique avec le trou d'évacuation (20) et appropriée pour empêcher la solution détergente ou tout fluide collecté dans le réservoir (2) de dépasser un niveau limite prédéterminé.

7. Tapis de désinfection (1) selon l'une quelconque des revendications 4 à 6, dans lequel le réservoir (2) loge une pluralité de modules de désinfection placés côte à côte de sorte que la surface de support (31) de chaque module de désinfection (3) repose sur le plan de support imaginaire (A), et
dans lequel le niveau de solution détergente contenue dans le réservoir (2) dépasse la hauteur du plan de support imaginaire (A), de sorte qu'uniquement la pluralité de faisceaux de poils (4) fassent saillie au-delà du plan de repos dudit plan de support imaginaire (A).

8. Tapis de désinfection (1) selon les revendications 6 et 7, dans lequel l'ouverture (25) est positionnée à une hauteur, mesurée verticalement à partir de la surface de marche (P), qui est supérieure à la hauteur à laquelle repose le plan de support imaginaire (A).

9. Tapis de désinfection (1) selon l'une quelconque des revendications 4 à 8, dans lequel chaque module de désinfection (3) est en forme de carreau et porte une grille de canaux (33, 35) pour canaliser la solution détergente, ladite grille de canaux (33, 35) étant également en communication fluidique avec la pluralité de fentes (5), de sorte que chaque faisceau de poils, soumis à la force de poids exercée par une semelle ou une roue, soit aplati et humidifié dans la solution détergente et exerce à la fois une action de nettoyage et une action de purification par brossage et/ou récurage.

10. Tapis de désinfection (1) selon la revendication précédente, dans lequel la grille de canaux est constituée sur la surface de support (31) de chaque module de désinfection (3) et comprend un premier groupe de canaux (33) orientés parallèlement les uns aux autres dans la première direction d'utilisation (D1) et/ou un deuxième groupe de canaux (35) agencés parallèlement les uns aux autres et orientés dans une deuxième direction d'utilisation (D2) incidente à la première direction d'utilisation (D1) ; de préférence lesdites première direction d'utilisation (D1) et deuxième direction d'utilisation (D2) étant orthogonales l'une à l'autre.

11. Tapis de désinfection (1) selon la revendication 9 ou 10, dans lequel, entre un module de désinfection (3) et le module de désinfection adjacent et/ou entre un module de désinfection (3) et la paroi latérale (22) du réservoir (2) se trouve un espacement (7) permettant à la solution de nettoyage d'atteindre la grille de canaux (33, 35) et la pluralité de fentes (5).

12. Tapis de désinfection (1) selon l'une quelconque des revendications précédentes, dans lequel la base de support (30) est constituée de POM-C.

13. Kit de désinfection (100) pour nettoyer des semelles et/ou des roues comprenant un tapis de désinfection (1) selon l'une quelconque des revendications 1 à 12 et une pluralité de modules de rampe (6), dans lequel chaque module de rampe de ladite pluralité de modules de rampe (6) est un plan incliné pour relier la surface de marche (P) au plan de support imaginaire (A).

14. Kit de désinfection (100) selon la revendication précédente en combinaison avec la revendication 6, comprenant en outre un outil de levage et de libération (8) pourvu de moyens de couplage (81) pouvant s'engager avec des contre-moyens de couplage (91) respectifs constitués sur chaque module de désinfection (3), sur chaque module de rampe de la pluralité de modules de rampe (6) et/ou sur l'extension (24).
